# EUROPEAN PATENT APPLICATION

(11) **EP 3 476 389 A1**
(43) Date of publication of application: **01.05.2019**
(21) Application number: 17306507.9
(22) Date of filing: 31.10.2017
(51) Int. Cl.: A61K 31/135, A61K 31/36, A61P 33/02

(54) **ADAMANTANE OR BICYCLIC MONOTERPENOID DERIVATIVES FOR USE IN THE TREATMENT OF LEISHMANIASIS**

(71) Applicant: Commissariat à l'Energie Atomique et aux Energies Alternatives, 75015 Paris (FR)
(72) Inventor: GILLET Daniel, 92160 ANTONY (FR); CINTRAT Jean-Christophe, 91430 IGNY (FR); BARBIER Julien, 91190 GIF-SUR-YVETTE (FR); PRUVOST Alain, 78460 CHOISEL (FR); LOISEAU Philippe, 91190 GIF-SUR-YVETTE (FR); VACUS Joël, 91530 SAINT MAURICE MONTCOURONNE (FR)
(74) Representative: Gevers & Orès

(57) **Abstract**

The present invention is in the field of therapeutic drugs to treat leishmaniasis. In particular, the invention concerns adamantane and bicyclic monoterpenoid derivatives for use in the treatment of leishmaniasis.

## Description

The present invention is in the field of therapeutic drugs to treat leishmaniasis. In particular, the invention concerns adamantane and bicyclic monoterpenoid derivatives for use in the treatment of leishmaniasis.

Leishmaniasis is a complex of tropical and sub-tropical diseases provoked by *Leishmania* protozoan parasites transmitted by the sand-fly vector and presenting different clinical expressions. There are three main forms of this disease: visceral, cutaneous and muco-cutaneous. Infections in humans are caused by more than 20 species of *Leishmania.* This disease affects 12 million humans with 500.000 new cases/year.

Dogs are considered as the major host for *Leishmania infantum* and the main reservoir for human infection in the Mediterranean basin. The clinical symptoms and the time of appearance of canine leishmaniasis vary from apparently healthy to critically diseased animals. Therefore early detection and treatment of infected animals are necessary to reduce the risk of infection in Humans. The prevalence of canine visceral leishmaniasis reaches 67-80% in enzootic Mediterranean regions. There are about 1 million cases of infected dogs in France, with 40,000 new cases each year. Total number of cases in southern Europe is about 2,5 million.

Cats are considered less prone to infection but a growing number of domestic cats are infected, either because of concurrent infection by immunodeficiency viruses or other unknown reasons.

Although the wild animal and livestock reservoirs of *Leishmania infantum* or other species of *Leishmania* are poorly understood, it has been show that farm animals (cattle, sheep, goat and donkey) are infected.

Regarding existing anti-leishmanial chemotherapy, Allopurinol is used to control the beginning of the infection. In constituted visceral leishmaniasis, dogs are treated with antimonials (Glucantime). Amphotericin B is too toxic to treat dogs and is reserved to Humans. In France, Miltefosine, the first orally active drug, is reserved to Humans and has no AMM for veterinary application.

However, even for human use, current drugs are highly toxic, aspecific, compliance of patients to treatment is low, as treatment is long and drug price is high. In addition, resistance is common, mainly to antimonials (Glucantime), the most classical drugs to treat dogs, to amphotericin B and now to Miltefosine.

There is thus an urgent need for new leishmaniasis drugs.

Accordingly, it is an object of the present invention to provide compounds alternative to conventional anti-leishmanial chemotherapy, in particular for the treatment of human and animal (including dog) leishmaniasis.

Inventors have for the first time demonstrated that a selection of adamantane and bicyclic monoterpenoid derivatives shows a strong anti-leishmaniasis activity.

In particular, the capacity of these compounds to block the development of intramacrophagic amastigote parasites has been tested.

Surprisingly, compounds of the invention are more toxic towards the intramacrophagic amastigotes than on the axenic amastigotes, more particularly at least 10 to 20 fold more toxic.

Without being bound to theory, it is believed that compounds of the invention act on the host by interfering with late endosomes that are hijacked by the parasites to build their parasitophorous vacuole.

In fact, molecules with a high inhibitory activity (low EC50) against intramacrophagic leishmania parasites and a low inhibitory activity (high IC50) against the axenic parasites represent an original approach to treat leishmaniasis. Drug's antiparasitic activity is due mainly to action on a host mechanism rather than direct action on the parasite. In contrast, current drugs such as allopurinol, glucantime (antimonials), amphotericin B and miltefosine act directly on the parasite.

Advantageously, it has been suggested that anti-infectious drugs acting on the host rather than on the pathogen are less likely to induce drug resistant pathogens.

### Compounds of formula (I) for use in the treatment of Leishmania infection

Thus, in one aspect, the present invention relates to a compound of formula (I): 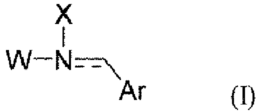 Wherein:
W is independently selected from:
   - an adamantyl optionally substituted by one or more C₁-C₆ alkyl groups,
   - a bicyclic monoterpene or a bicyclic monoterpenoid, in particular:
      ∘ a pinanyl,
      ∘ a bornanyl, or
      ∘ an isopinocamphenyl,
W being optionally substituted by one to three OH groups,
-̅-̅-̅-̅ represents a single bond or a double bond,
X is Ø or selected from H and C₁-C₆ alkyl groups, providing that:
   - X is Ø when -̅-̅-̅-̅ represents a double bond, and
   - X is selected from H and C₁-C₆ alkyl groups when -̅-̅-̅-̅ represents a single bond,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, OH, C₁-C₆ alkoxy, NO₂, C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, OH, C₁-C₆ alkoxy and NO₂,
or said aryl or heteroaryl group Ar optionally forming with said aryl or heteroaryl group R a tricyclic compound, in particular a fluorenyl,
and the stereoisomeric forms, mixtures of stereoisomeric forms or pharmaceutically acceptable salts forms thereof,
for use in the treatment of leishmaniasis.

The present invention also relates to a compound of formula (I): 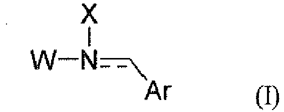 Wherein:
W is independently selected from:
   - an adamantyl optionally substituted by one or more C₁-C₆ alkyl groups,
   - a pinanyl,
   - a bornanyl, or
   - an isopinocatnphenyl,
-̅-̅-̅-̅ represents a single bond or a double bond,
X is Ø or selected from H and C₁-C₆ alkyl groups, providing that:
   - X is Ø when -̅-̅-̅-̅ represents a double bond, and
   - X is selected from H and C₁-C₆ alkyl groups when -̅-̅-̅-̅ represents a single bond,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, C₁-C₆ alkoxy, NO₂, C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, C₁-C₆ alkoxy and NO_{2,} or said aryl or heteroaryl group Ar optionally forming with said aryl or heteroaryl group R a tricyclic compound, in particular a fluorenyl,
and the stereoisomeric forms, mixtures of stereoisomeric forms or pharmaceutically acceptable salts forms thereof,
for use in the treatment of leishmaniasis.

In another embodiment, there are included compounds of formula (I) for use as defined above, wherein:
W is independently selected from:
   - an adamantyl optionally substituted by one or more C₁-C₆ alkyl groups,
   - a pinanyl,
   - a barnanyl, or
   - an isopinocamphenyl,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, OH, C₁-C₆ alkoxy, NO_{2,} C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, OH, C₁-C₆ alkoxy and NO_{2,}
or said aryl or heteroaryl group Ar optionally forming with said aryl or heteroaryl group R a tricyclic compound, in particular a fluorenyl.

In a particular embodiment, there are included compounds of formula (I) for use as defined above, wherein said Ar is substituted by one to three OH groups, in particular one or two OH groups, said Ar being optionally further substituted by one to three R groups, R is independently selected from F, Cl, Br, I, C₁-C₆ alkyl, OH, C₁-C₆ alkoxy, NO_{2,} C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, OH, C₁-C₆ alkoxy and NO_{2,} said .Ar being more particularly a phenyl group.

In another embodiment, there are included compounds of formula (I) for use as defined above, wherein:
W is independently selected from:
   - an adamantyl optionally substituted by one or more C₁-C₆ alkyl groups,
   - a pinanyl,
   - a bornanyl, or
   - an isopinocamphenyl,
W being optionally substituted by one to three OH groups,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, C₁-C₆ alkoxy, NO_{2,} C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, C₁-C₆ alkoxy and NO_{2,} or said aryl or heteroaryl group Ar optionally forming with said aryl or heteroaryl group R a tricyclic compound, in particular a fluorenyl.

In a particular embodiment, there are included compounds of formula (I) for use as defined above, wherein W is substituted by one to three OH groups, in particular one or two OH groups, said W being more particularly an adamantyl optionally substituted by one or more C₁-C₆ alkyl groups.

In a particular embodiment, there are included compounds of formula (I) for use as defined above, to block or slow down the development of intramacrophagic amastigote parasites.

In another embodiment, there are included compounds of formula (I) for use as defined above, being of one of the following formulae: 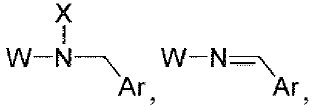 X being in particular H or Me.

In another embodiment, there are included compounds of formula (I) for use as defined above, wherein Ar is a C₆-C₁₀ aryl, notably phenyl, or wherein Ar and a R group form together a fluorenyl.

In another embodiment, there are included compounds of formula (I) for use as defined above, wherein Ar is a 5 to 10 membered heteroaryl, notably a furanyl, an imidazolyl or an indolyl group.

In another embodiment, there are included compounds of formula (I) for use as defined above, wherein R is selected from Br, Cl, I, methoxy, NO₂, imidazolyl and R'-phenyl, in particular p-methyl phenyl.

In another embodiment, there are included compounds of formula (I) for use as defined above, wherein W is an adamantyl optionally substituted by one or more C₁-C₆ alkyl groups, in particular of the following formula: 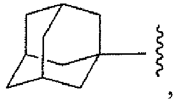 W being optionally substituted by one to three OH groups,
Ar being in particular a phenyl, being optionally substituted by one to three R groups, notably two R groups, in particular independently selected from F, Cl, Br, I, C₁-C₆ alkyl, C₁-C₆ alkoxy and NO₂, more particularly Br and methoxy.

In another embodiment, there are included compounds of formula (I) for use as defined above, wherein W is a pinanyl, a bornanyl or an isopinocamphenyl, in particular of the following formula: 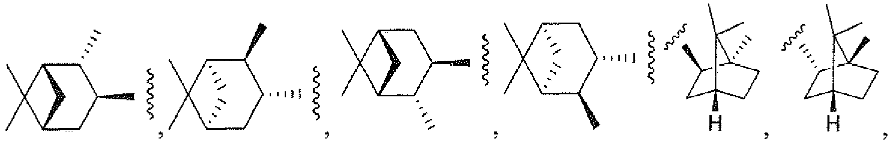 W being optionally substituted by one to three OH groups.

In another embodiment, there are included compounds of formula (I) for use as defined above, providing that when Ar represents a phenyl substituted by an halogen, in particular Br, and a C₁-C₆ alkoxy, in particular a methoxy, more particularly a 5-bromo-2-methoxyphenyl group, then:
- -̅-̅-̅-̅ represents a double bond, or
- X represents a C₁-C₆ alkyl group, or
- W represents a pinanyl, a bornanyl or an isopinocamphenyl, in particular a pinanyl, W being optionally substituted by one to three OH groups.

In another embodiment, there are included compounds of formula (I) for use as defined above, providing that:
- -̅-̅-̅-̅ represents a double bond, or
- X represents a C₁-C₆ alkyl group, or
- W represents a pinanyl, a bornanyl or an isopinocamphenyl, in particular a pinanyl, W being optionally substituted by one to three OH groups.

In another embodiment, there are included compounds of formula (I) for use as defined above, providing that Ar is not a phenyl substituted by an halogen, in particular Br, and a C₁-C₆ alkoxy, in particular a methoxy, more particularly a 5-bromo-2-methoxyphenyl group, and that:
- -̅-̅-̅-̅ represents a double bond, or
- X represents a C₁-C₆ alkyl group, or
- W represents a pinanyl, a bornanyl or an isopinocamphenyl, in particular a pinanyl, W being optionally substituted by one to three OH groups.

In another embodiment, there are included compounds of formula (I) for use as defined above, which are selected from:

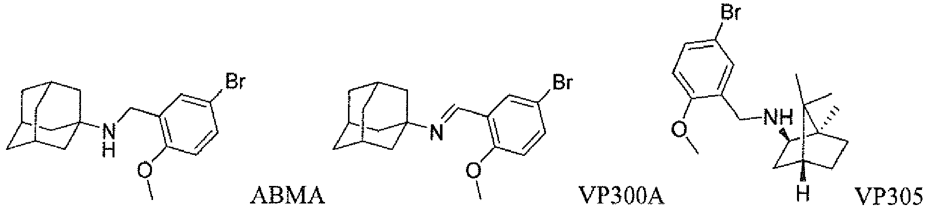

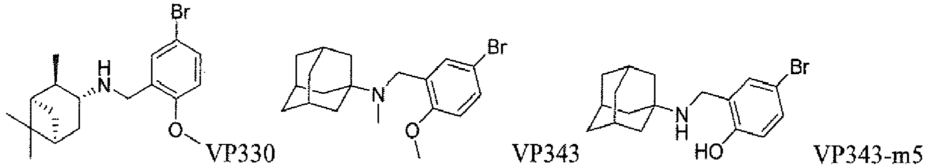

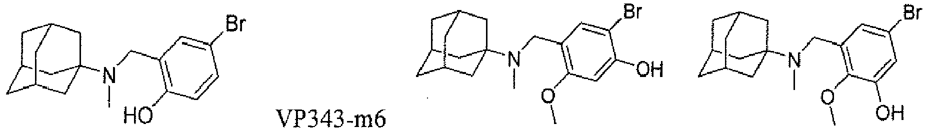

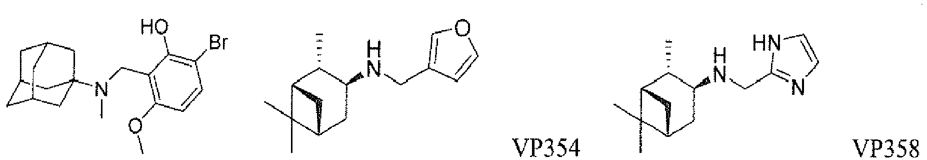

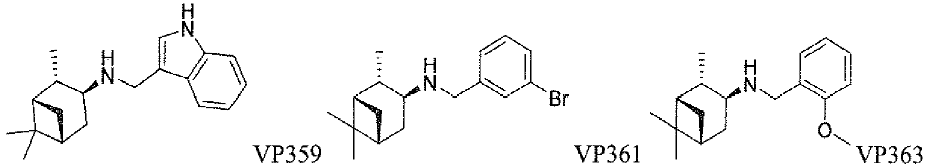

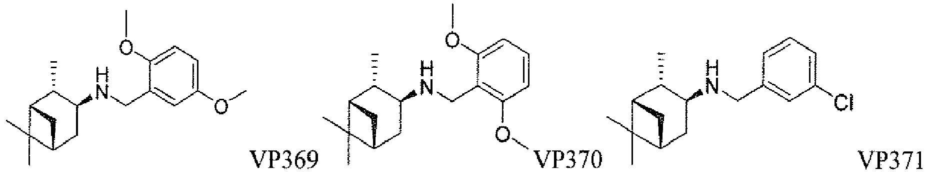

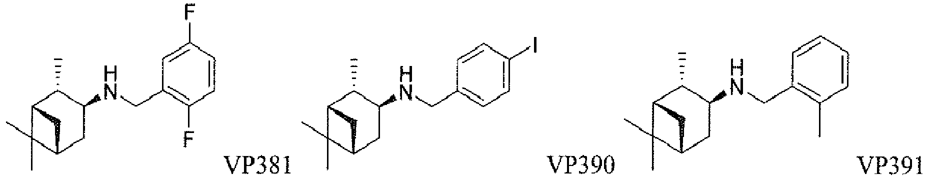

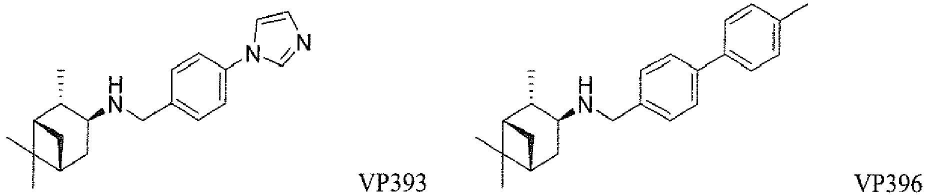

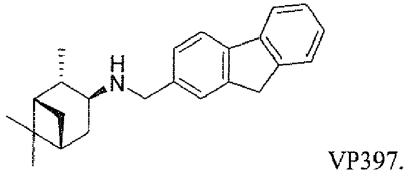

In another embodiment, there are included compounds of formula (I) for use as defined above, which are selected from:

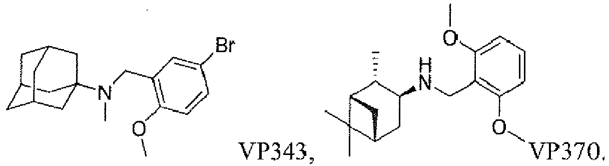

In another embodiment, there are included compounds of formula (I) for use as defined above, said compounds being different from:

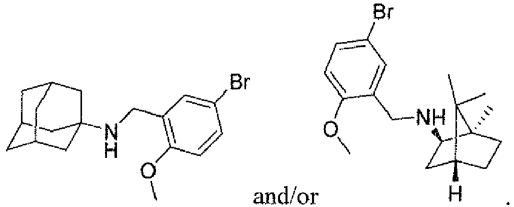

In another embodiment, there are included compounds of formula (I) for use as defined above, for the treatment of mammal leishmaniasis.

In another embodiment, there are included compounds of formula (I) for use as defined above, for the treatment of human leishmaniasis, for example the treatment of leishmaniasis of the human child.

In another embodiment, there are included compounds of formula (I) for use as defined above, for the treatment of animal leishmaniasis, in particular dogs, cats, horses, lagomorphs, notably hares and rabbits, rodents, in particular mice and rats, and farm animals, notably Alpaca, Buffalo, Banteng, Camel, Cow, Chicken, Duck, Emu, Goat, Goose, Llama, Pig, Pigeon, Rhea, Rabbit, Sheep, Turkey, Yak or Zebu.

According to one embodiment, the present invention relates to compounds of formula (I) for their use for the treatment of visceral, cutaneous and/or muco-cutaneous leishmaniasis.

According to one embodiment, the present invention relates to compounds of formula (I) for their use for the treatment of leishmaniasis caused by trypanosomes of the genus *Leishmania,* in particular of:
- The subgenus Leishmania, more particularly:
   - *Leishmania aethiopica*
   - *Leishmania amazonensis*
   - *Leishmania arabica*
   - *Leishmania donovani*
   - *Leishmania gerbilli*
   - *Leishmania hertigi*
   - *Leishmania infantum*
   - *Leishmania infantum*/*donovani*
   - *Leishmania killicki*
   - *Leishmania major*
   - *Leishmania mexicana*
   - *Leishmania siamensis*
   - *Leishmania tropica*
   - *Leishmania turanica*
- The subgenus Sauroleishmania, more particularly:
   - *Leishmania adleri*
   - *Leishmania agamae*
   - *Leishmania ceramodactyli*
   - *Leishmania deanei*
   - *Leishmania garnhami*
   - *Leishmania gulikae*
   - *Leishmania gymnodactyli*
   - *Leishmania hemidactyli*
   - *Leishmania hoogstraali*
   - *Leishmania nicollei*
   - *Leishmania senegalensis*
   - *Leishmania tarentolae*
- The subgenus Viannia, more particularly:
   - *Leishmania braziliensis*
   - *Leishmania colombiensis*
   - *Leishmania equatorensis*
   - *Leishmania guyanensis*
   - *Leishmania lainsoni*
   - *Leishmania naiffi*
   - *Leishmania panamensis*
   - *Leishmania peruviana*
   - *Leishmania pifanoi*
   - *Leishmania shawi*
   - *Leishmania utingensis*
or trypanosomes of the genus *Endotrypanum,* in particular *Endotrypanum monterogeii* or *Endotrypanum schaudinni,* or trypanosomes of the *L. enrittii* complex, in particular *Leishmania enrittii* or *Leishmania martiniquensis.*

According to one embodiment, the present invention relates to compounds of formula (I) for their use for the treatment of leishmaniasis caused by *Leishmania infantum, Leishmania donovani or Leishmania infantum*/*donovani hybrid.*

### Pharmaceutical compositions

In a second aspect, the present invention relates to a pharmaceutical composition comprising a compound of formula (Ia): 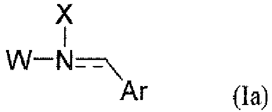 Wherein:
W is independently selected from:
   - an adamantyl optionally substituted by one or more C₁-C₆ alkyl groups, or
   - a pinanyl or an isopinocamphenyl, in particular 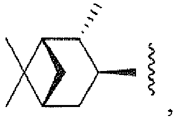 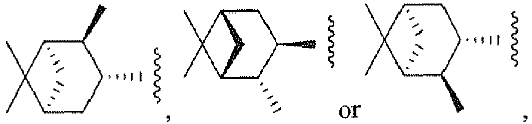
W being optionally substituted by one to three OH groups,
-̅-̅-̅-̅ represents a single bond or a double bond,
X is Ø or selected from H and C₁-C₆ alkyl groups, providing that:
   - X is Ø when -̅-̅-̅-̅ represents a double bond, and
   - X is selected from H and C₁-C₆ alkyl groups when -̅-̅-̅-̅ represents a single bond,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, OH, C₁-C₆ alkoxy, NO_{2,} C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, OH, C₁-C₆ alkoxy and NO_{2,} or said aryl or heteroaryl group Ar optionally forming with said aryl or heteroaryl group R a tricyclic compound, in particular a fluorenyl, providing that:
   - When W is an adamantyl optionally substituted by one or more C₁-C₆ alkyl groups, then:
      ∘ X is selected from C₁-C₆ alkyl groups, Ar being a phenyl substituted by an halogen, in particular Br, and a C₁-C₆ alkoxy, in particular a methoxy, or
      ∘ X is Ø, Ar being a phenyl substituted by an halogen, in particular Br, and a C₁-C₆ alkoxy, in particular a methoxy,
   - When W is 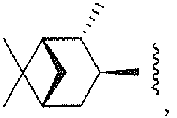 then:
      ∘ Ar is an indolyl optionally substituted by one to three R groups, or
      ∘ Ar is a substituted phenyl different from 5-bromo-2-methoxyphenyl, 5-iodo-2-methoxyphenyl, 4-iodophenyl and 4-fluorophenyl, and Ar being optionally different from 4-methylphenyl,
and the stereoisomeric forms, mixtures of stereoisomeric forms or pharmaceutically acceptable salts forms thereof,
in admixture with one or more pharmaceutically acceptable excipients.

It is to be noted that the present invention includes the combination of all the embodiments listed hereabove for formula (I).

In a particular embodiment, there are included pharmaceutical compositions comprising a compound of formula (Ia), of particular formula (Ib): 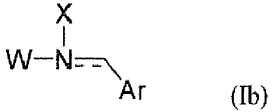 Wherein:
W is independently selected from:
   - an adamantyl optionally substituted by one or more C₁-C₆ alkyl groups,
   - a pinanyl,
   - a bornanyl, or
   - an isopinocamphenyl,
W being optionally substituted by one to three OH groups, -̅-̅-̅-̅
represents a single bond or a double bond,
X is Ø or selected from H and C₁-C₆ alkyl groups, providing that:
   - X is Ø when -̅-̅-̅-̅ represents a double bond, and
   - X is selected from H and C₁-C₆ alkyl groups when -̅-̅-̅-̅ represents a single bond,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, OH, C₁-C₆ alkoxy, NO_{2,} C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, OH, C₁-C₆ alkoxy and NO_{2,}
or said aryl or heteroaryl group Ar optionally forming with said aryl or heteroaryl group R a tricyclic compound, in particular a fluorenyl,
providing that:
   - X is selected from C₁-C₆ alkyl groups, or
   - Ar is substituted by one to three R groups, at least one of them being a C₆-C₁₀ aryl or a 5 to 10 membered heteroaryl optionally substituted by one to three R' groups as defined above, said Ar optionally forming with said R group a tricyclic compound, in particular a fluorenyl,
and the stereoisomeric forms, mixtures of stereoisomeric forms or pharmaceutically acceptable salts forms thereof,
in admixture with one or more pharmaceutically acceptable excipients.

In a particular embodiment, there are included pharmaceutical compositions comprising a compound of formula (Ia) or (Ib), wherein Ar is substituted by one to three R groups as defined above, at least one of them being a C₆-C₁₀ aryl or a 5 to 10 membered heteroaryl optionally substituted by one to three R' groups as defined above.

In another embodiment, there are included pharmaceutical compositions comprising a compound of formula (Ia) or (Ib) as defined above,
said compound being of one of the following formulae: 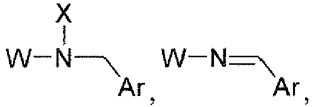 X being in particular H or Me.

In another embodiment, there are included pharmaceutical compositions comprising a compound of formula (Ia) or (Ib) as defined above, wherein Ar is a C₆-C₁₀ aryl, notably phenyl, or wherein Ar and a R group form together a fluorenyl.

In another embodiment, there are included pharmaceutical compositions comprising a compound of formula (Ia) or (Ib) as defined above, wherein Ar is a 5 to 10 membered heteroaryl, notably a furanyl, an imidazolyl or an indolyl group.

In another embodiment, there are included pharmaceutical compositions comprising a compound of formula (Ia) or (Ib) as defined above, wherein R is selected from Br, Cl, I, methoxy, NO₂, imidazolyl and R'-phenyl, in particular p-methyl phenyl.

In another embodiment, there are included pharmaceutical compositions comprising a compound of formula (Ia) or (Ib) as defined above, wherein W is an adamantyl optionally substituted by one or more C₁-C₆ alkyl groups, in particular of the following formula: 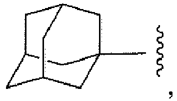 W being optionally substituted by one to three OH groups,
Ar being in particular a phenyl, being optionally substituted by one to three R groups, notably two R groups, in particular independently selected from F, Cl, Br, I, C₁-C₆ alkyl, C₁-C₆ alkoxy and NO₂, more particularly Br and methoxy.

In another embodiment, there are included pharmaceutical compositions comprising a compound of formula (Ia) or (Ib) as defined above, wherein W is a pinanyl, a bornanyl or an isopinocamphenyl, in particular of the following formula: 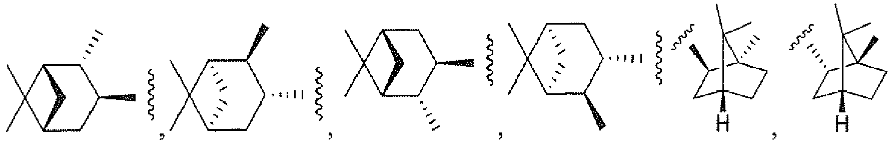 W being optionally substituted by one to three OH groups.

In another embodiment, there are included pharmaceutical compositions comprising a compound of formula (Ia) as defined above, which are selected from:

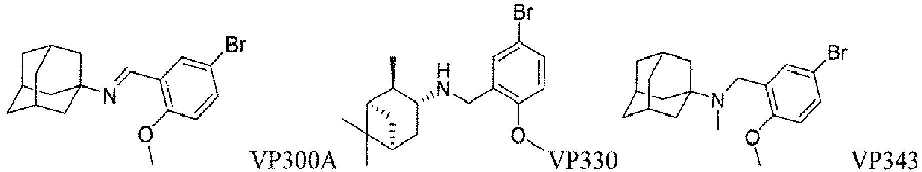

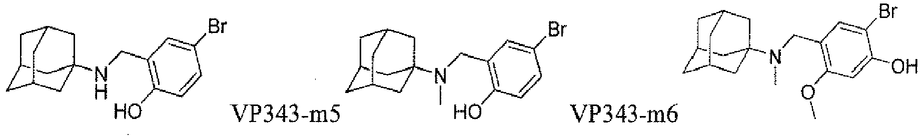

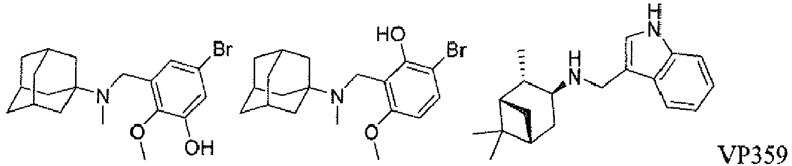

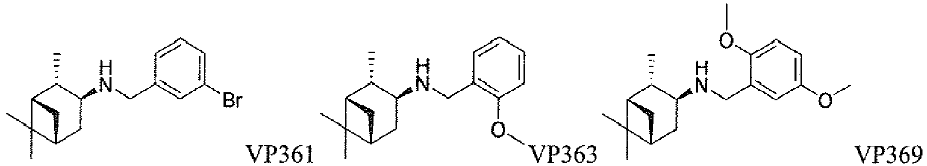

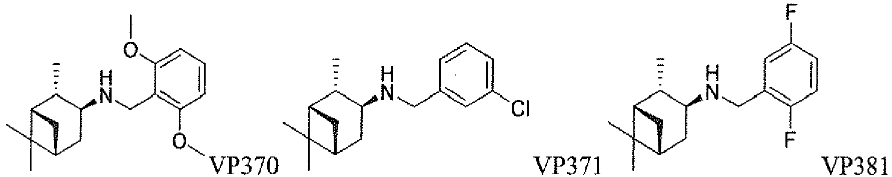

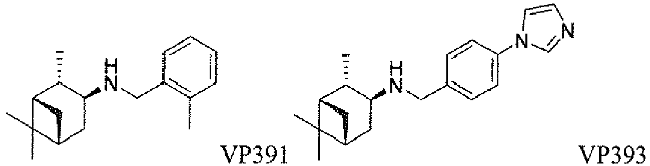

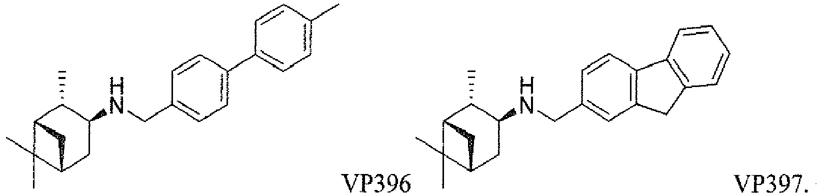

In another embodiment, there are included pharmaceutical compositions comprising a compound of formula (Ia) or (Ib) as defined above, which are selected from:

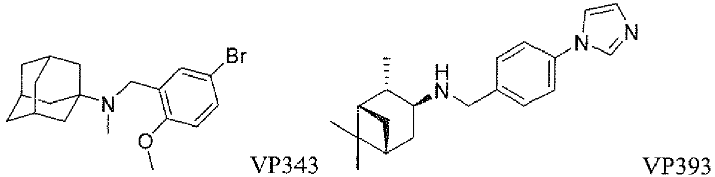

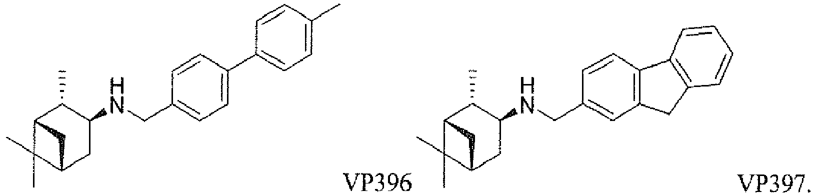

In another embodiment, there are included pharmaceutical compositions comprising a compound of formula (Ia) or (Ib) as defined above, which are selected from:

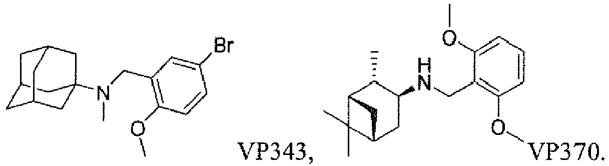

The compounds of formula (I) and/or (Ia) and/or (Ib) of the present invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

The compounds of formula (I) and/or (Ia) and/or (Ib) of the present invention may in particular be administered topically, notably in the form of a gel.

The compounds of formula (I) and/or (Ia) and/or (Ib) of the present invention may in particular be administered topically, more particularly on the skin or on mucosa lesions.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of the present invention, inert, pharmaceutically acceptable carriers can be either solid, gel or liquid. Solid form preparations include powders, tablets, dispersible granules, capsules, cachets, and suppositories.

In particular, the pharmaceutical composition is a topical formulation.

The term "topical formulation" refers to a composition formulated such that the active ingredient(s) of the composition may be placed for direct application to a skin surface and from which an effective amount of the active ingredient(s) is released. Examples of topical formulations include, but are not limited to, ointments, creams, gels, lotions, sprays, pastes, and the like. In certain embodiments of the present invention, the compositions are formulated as creams or gels.

In particular, the pharmaceutical composition is an injectable formulation.

The term "injectable formulation" refers to an injectable composition, such as in the form of a fluid, semi-solid or gel-like substance. The term "injectable" means that the formulation is engageable and/or insertable into or onto a desired location of the body of an individual or patient, preferably by being able to flow under the application of a sufficient pressure, in particular through a needle or catheter or other suitable applicator for the application of fluids, semi-solids or gel-like substances.

A solid carrier can be one or more substances, which may also act as diluents, flavouring agents, solubilizers, lubricants, suspending agents, binders, or tablet disintegrating agents; it can also be an encapsulating material.

Tablets, powders, cachets, and capsules can be used as solid dosage forms suitable for oral administration.

Liquid form compositions include solutions, suspensions, and emulsions. For example, sterile water or propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavouring agents, stabilizers, and thickening agents as desired. Aqueous solutions for oral use can be made by dispersing the finely divided active component in water together with a viscous material such as natural synthetic gums, resins, methylcellulose, sodium carboxymethyl cellulose, and other suspending agents known to the pharmaceutical formulation art.

In a particular embodiment, the pharmaceutical composition comprises a polysorbate, more particularly a polysorbate 80 (e.g. Tween 80).

Depending on the mode of administration, the pharmaceutical composition will according to one embodiment of the present invention include 0.05% to 99% weight (percent by weight), according to an alternative embodiment from 0.10 to 50% weight, of the compound of the present invention, all percentages by weight being based on total composition. A therapeutically effective amount for the practice of the present invention may be determined, by the use of known criteria including the age, weight and response of the individual patient, and interpreted within the context of the disease which is being treated or which is being prevented, by one of ordinary skills in the art.

### Compounds of formula (Ia)

In a third aspect, the present invention relates to a compound of formula (Ia) or (Ib) as defined above, and the stereoisomeric forms, mixtures of stereoisomeric forms or pharmaceutically acceptable salts forms thereof.

It is to be noted that the present invention includes the combination of all the embodiments listed hereabove for formulae (I) or (Ia) or (Ib), respectively.

### Definitions

The following terms and expressions contained herein are defined as follows:

As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein, the term "alkyl" refers to a straight-chain, or branched alkyl group having 1 to 8 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, octyl, etc. The alkyl moiety of alkyl-containing groups, such as alkoxy, alkoxycarbonyl, and alkylaminocarbonyl groups, has the same meaning as alkyl defined above. Lower alkyl groups, which are preferred, are alkyl groups as defined above which contain 1 to 4 carbons. A designation such as "C₁-C₄ alkyl" refers to an alkyl radical containing from 1 to 4 carbon atoms.

As used herein, the term "alkoxy" refers to an -O-alkyl group, wherein the term alkyl is as defined herein. Examples of alkoxy groups notably include methoxy, ethoxy, n-propoxy groups.

As used herein, the term "aryl" refers to a substituted or unsubstituted, mono- or bicyclic hydrocarbon aromatic ring system having 6 to 10 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

As used herein, the term "heteroaryl" refers to an aromatic group containing 5 to 10 ring carbon atoms in which one or more ring carbon atoms are replaced by at least one hetero atom such as -O-, -N-, or -S-. Examples of heteroaryl groups include pyrrolyl, furanyl, thienyl, pirazolyl, imidazolyl, thiazolyl, isothiazolyl, isoxazolyl, oxazolyl, oxathiolyl, oxadiazolyl, triazolyl, oxatriazolyl, furazanyl, tetrazolyl, pyridyl, pyrazinyl, pyrimidinyl, pyridazinyl, triazinyl, indolyl, isoindolyl, indazolyl, benzofuranyl, isobenzofuranyl, purinyl, quinazolinyl, quinolyl, isoquinolyl, benzoimidazolyl, benzothiazolyl, benzothiophenyl, thianaphthenyl, benzoxazolyl, benzisoxazolyl, cinnolinyl, phthalazinyl, naphthyridinyl, and quinoxalinyl. Included within the definition of "heteroaryl" are fused ring systems, including, for example, ring systems in which an aromatic ring is fused to a heterocycloalkyl ring. Examples of such fused ring systems include, for example, phthalamide, phthalic anhydride, indoline, isoindoline, tetrahydroisoquinoline, chroman, isochroman, chromene, and isochromene.

As used herein, a "therapeutically effective amount" refers to an amount of a compound of the present invention effective to prevent or treat the symptoms of particular disorder.

As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

By "monoterpene" is meant any terpene of formula C₁₀H₁₆, i.e. that contains two isoprene units.

By "monoterpenoid" is meant a modified monoterpene, said modification being in particular an oxidation, a rearrangement and/or the saturation of a double bond.

All other terms used in the description of the present invention have their meanings as is well known in the art.

In another aspect, the present invention is directed to pharmaceutically acceptable salts of the compounds described above. As used herein, "pharmaceutically acceptable salts" includes salts of compounds of the present invention derived from the combination of such compounds with non-toxic acid or base addition salts.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.

Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

In addition to pharmaceutically-acceptable salts, other salts are included in the invention. They may serve as intermediates in the purification of the compounds, in the preparation of other salts, or in the identification and characterization of the compounds or intermediates.

The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent. Such solvates are within the scope of the present invention.

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

### Synthesis

The compounds of the present invention may be prepared in a number of methods well known to those skilled in the art, including, but not limited to those described below, or through modifications of these methods by applying standard techniques known to those skilled in the art of organic synthesis. The appropriate modifications and substitutions will be readily apparent and well known or readily obtainable from the scientific literature to those skilled in the art. In particular, such methods can be found in R.C. Larock, Comprehensive Organic Transformations, Wiley-VCH Publishers, 1999.

All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

The general routes to prepare the examples of the present invention involve in particular an amine of formula W-NH₂ and an aldehyde of formula Ar-COH, through imine formation or reductive amination, optionally followed by the alkylation of the obtained amine. The reagents and starting materials are commercially available, or readily synthesized by well-known techniques by one of ordinary skill in the arts. All substituents in the synthetic Schemes, unless otherwise indicated, are as previously defined.

### DESCRIPTION OF THE DRAWINGS

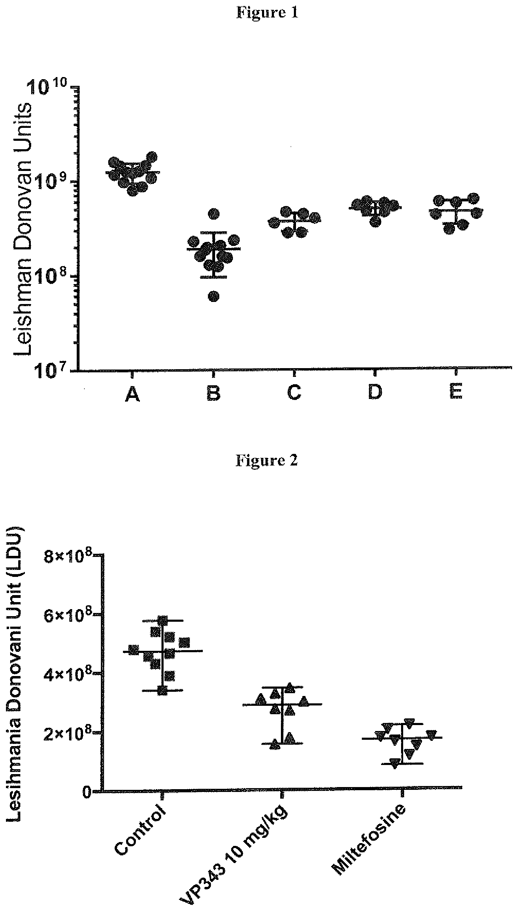

**Figure 1** shows the counts (LDU) of parasites in the liver of mice infected and treated with VP343 (batches C and D), VP370 (batch E), with Miltefosine as reference molecule (batch B), or untreated (batch A). Experimental conditions tested are presented in table III and the counts determination is described in the protocol of example 5. p<0.0001 for all experimental groups versus untreated control.
**Figure 2** shows the counts (LDU) of parasites in the liver of mice infected and treated with VP343 at 10 mg/kg or Miltefosine at 10 mg/kg as reference molecule or untreated. Experimental conditions tested are presented in table V and the counts determination is described in the protocol of example 5. Control versus VP343: p= 0.0235. Control versus Miltefosine: p<0.0001.

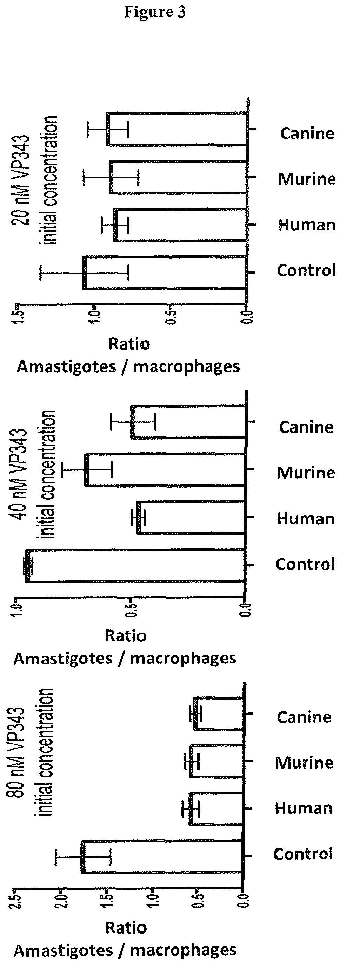

**Figure 3** shows the inhibition of growth of intramacrophage amastigote leishmania parasites by supernatants from mouse, dog and human hepatic microsomes incubated with VP343 for 45 min. The IC₅₀ of VP343 is 40 nM (*cf.* Table II). The indicated VP343 initial concentrations correspond to a concentration equivalent of VP343 at t = 0 obtained after dilution of the supernatants.

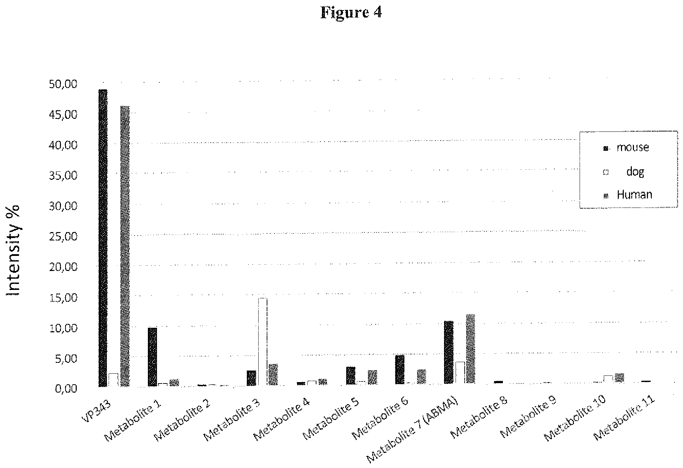

**Figure 4** shows the LC-MS peak intensities % (expressed as ratio of compound's intensity to VP343 intensity at t = 0) of VP343 and its metabolites identified in supernatants from mouse, dog and human hepatic microsomes incubated with VP343 for 45 min.

### EXAMPLES

### Example 1 : Representative procedure for the synthesis of VP300A

Under nitrogen, a 100 mL flame dried round bottom flask equipped with a stirring bar is charged with 6.5 mmol (981 mgs) of 1-adamantylamine and 50 mL of anhydrous methanol. 3-Bromo, 6-methoxybenzaldehyde (6.5 mmol) is then added and the solution is stirred overnight at room temperature. MeOH was then evaporated to dryness and the resulting mixture was dissolved in CH₂Cl₂ (100 mL), the organic phase was washed with 5% NaHCO₃ solution (50 mL), dried over MgSO₄, filtered and evaporated to dryness to afford VP300A (2 g, 88%) as a white solid.

### Example 2 : Representative procedure for the synthesis of VP343

In a 100 mL flame dried round bottom flask equipped with a stirring bar is placed 5 mmol (1.075 g) of 3-bromo,6-methoxybenzaldehyde. 40 mL of anhydrous methanol is then added. Under nitrogen is added 5 mmol (0.755 g) of 1-adamantylamine. After 4 hours of gentle agitation, 6 mmol of NaBH₃CN are added at once, followed by dropwise addition of glacial acetic acid (10 mmol, 570 µL). The reaction mixture was allowed to stir overnight. The MeOH was evaporated and the resulting mixture was dissolved in CH₂Cl₂ (100 mL), the organic phase was washed with 5% NaHCO₃ solution (50 mL), 1M HCl (50 mL), water (50 mL), saturated NaCl solution (50 mL). The organic phase was dried on MgSO₄, filtered and evaporated to dryness. The resulting oil was dissolved in 100 mL of anhydrous THF, 10 mmol of NaH (60% in mineral oil) was added at 0°C and 10 minutes later 5 mmols of MeI dropwise at 0°C. The solution was allowed to warm up to room temperature and was heated at 80°C overnight. The mixture was cooled down to 0°C and ice/water was carefully added. THF was evaporated, the mixture was dissolved in CH₂Cl₂ and purified by flash chromatography to give VP343 as a white solid (1.45 g).

### Example 3 : Representative procedure for the synthesis of VP370

In a 100 mL flame dried round bottom flask equipped with a stirring bar is placed 5 mmol (830 mg) of 2,6-dimethoxybenzaldehyde. 40 mL of anhydrous methanol is then added. Under nitrogen is added 5 mmol of (+)-isopinocampheylamine. The resulting solution slightly turns to pale yellow. After 4 hours of gentle agitation, 7.5 mmol of NaBH₃CN (470 mg) is added at once, followed by dropwise addition of glacial acetic acid (10 mmol, 570 µL). The reaction mixture turns more yellow and was allowed to stir overnight. The MeOH was evaporated and the resulting mixture was dissolved in CH₂Cl₂ (100 mL), the organic phase was washed with 5% NaHCO₃ solution (50 mL), 1M HCl (50 mL), water (50 mL), saturated NaCl solution (50 mL). The organic phase was dried on MgSO₄, filtered and evaporated to dryness. The resulting oil was taken up in 2M HCl/Et₂O (5 mL) and evaporated to dryness to afford 1.2 g of VP370 as a white amorphous solid. Compounds VP305, VP330, VP354, VP358, VP359, VP361, VP363, VP369, VP371, VP381, VP390, VP391, VP393, VP396 and VP397 were synthesized accordingly.

### Example 4 : In vitro evaluation of compounds of the invention

### Protocol for evaluation on Leishmania donovani infantum axenic amastigotes

Amastigote forms of the parasite were suspended to yield 10⁷ parasitic cells mL⁻¹. The maximum final compound concentration used was 100 µM. Triplicates were used for each concentration. Cultures were incubated at 37 °C for 72 h in the dark and under a 5% CO₂ atmosphere, then the viability of the amastigotes was assessed using the SYBR® Green I (Invitrogen, France) incorporation method. Parasite growth was determined by using SYBR® Green I, a dye with marked fluorescence enhancement upon contact with parasite DNA. Parasites were lysed and quantified by Direct PCR-Cell Genotyping without DNA isolation protocol (Euromedex, France). 10 µL of lysed parasite solution from each well was added to 40 µL of PCR-Cell reagent containing the SYBR® green I in a qPCR plate of 96 wells, and the contents were mixed. Fluorescence was measured with Mastercycler® ep realplex (Eppendorf, France). Measured fluorescence was compared to that from the range obtained with different parasite densities. The antileishmanial activity was expressed as IC₅₀ in µM (concentration of drug inhibiting 50% of the parasite growth, comparatively to the controls treated with the excipient only).

### Protocol for evaluation on the L. donovani infantum / RAW 264.7 macrophage model (intramacrophagic amastigotes)

Mouse macrophage cells RAW 264.7 (ATCC) were seeded into a 96-well microplate and maintained in DMEM medium (Applied Biosystems, France) supplemented with 10% heat-inactivated fetal bovine serum in a 5% CO₂ incubator at 37°C for 24 h. Then, the medium was replaced with 100 µL fresh medium containing a suspension of 10⁵ *L. infantum* amastigotes and the incubation time was 16 h. Then, extracellular parasites were eliminated from the medium and 100 µL fresh medium containing compound in various concentrations were added for a 48 h-incubation. Parasite growth was determined by using SYBR® Green I (Life technology, Fisher Scientific, France), a dye with marked fluorescence enhancement upon contact with DNA. The cell lysis suspension was diluted 1:1 in lysis buffer (10 mM NaCl, 1 mM Tris HCl pH8, 2.5 mM EDTA pH 8, 0.05% SDS, 0.01 mg/mL proteinase K and 10 × SYBR Green I). Incorporation of SYBR Green I in DNA amplification was measured using the Master epRealplex cycler® (Eppendorf, France) according to the following program to increase the SYBR green incorporation: 90°C for 1 min, decrease in temperature from 90°C to 10°C for 5 min with reading the fluorescence at 10°C for 1 min and a new reading at 10°C for 2 min. The EC₅₀ as the concentration inhibiting the parasite growth by 50% was calculated by nonlinear regression using icestimator website 1.2 version: http://www.antimalarial-icestimator.net/MethodIntro.htm.

### Results

Results concerning ABMA are presented in following table I.

**Table I. in vitro activities of ABMA and reference molecules on axenic and intramacrophagic amastigotes.**

| **Compound** | **Axenic amastigotes IC₅₀(µM)** | **Intramacrophagic amastigotes IC₅₀(µM)** | **Cytotoxicity on RAW 264.7 macrophag es CC₅₀(µM)** | **Selectivity IC₅₀ (Axenic amastigotes)/IC₅₀ (Intra macrophagic amastigotes)** |
|---|---|---|---|---|
| **ABMA** | >100 | 7.1 ± 1.7 | 25.3 ± 2.4 | >14 |
| **Miltefosine** | 1.2 ± 0.5 | 0.85 ± 0.20 | 12.5 ± 1.3 | 1.4 |
| **Amphotericin B** | 0.031 ± 0.002 | 0.047 ± 0.005 | 4.5 ± 0.4 | <1 |

These results strongly suggest that ABMA blocks *L. infantum* intracellular development by an action on the macrophage while the other drugs are directly toxic to the parasite.

Results concerning ABMA and other compounds of the invention are presented in following Table II.

**Table II. In vitro evaluation of compounds on Leishmania infantum axenic amastigotes and intramacrophagic amastigotes.**

| **Compound** | **Structure** | **IC₅₀ axenic (µM)** | **IC₅₀ intramacrophagic (µM)** |
|---|---|---|---|
| ABMA | 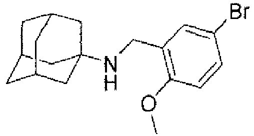 | > 100 | 7.10 |
| VP300A | 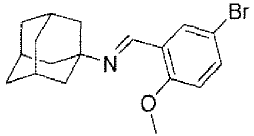 | 4.53 | 0.21 |
| VP305 | 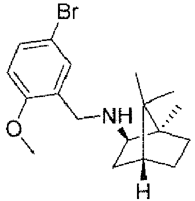 | < 0.5 | 0.06 |
| VP330 | 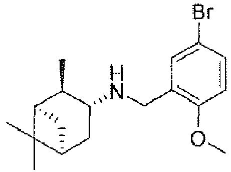 | 4.86 | 0.25 |
| VP343 | 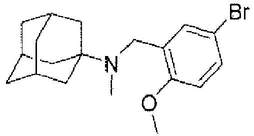 | 9.29 | 0.04 |
| VP354 | 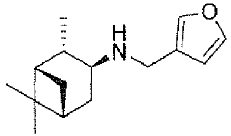 | 4.10 | 0.42 |
| VP358 | 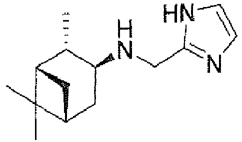 | 12.95 | 0.57 |
| VP359 | 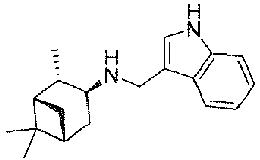 | > 100 | 0.25 |
| VP361 | 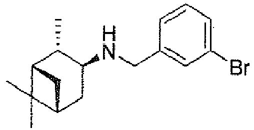 | 3.70 | 0.39 |
| VP363 | 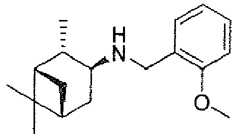 | 9.99 | 0.19 |
| VP369 | 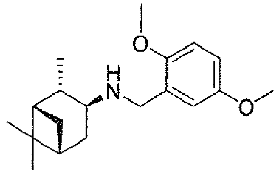 | 15.45 | 0.11 |
| VP370 | 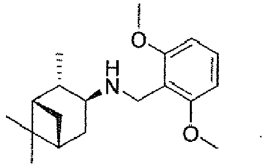 | 36.27 | 0.04 |
| VP371 | 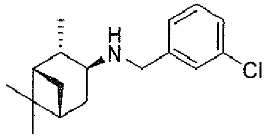 | 30.54 | 0.19 |
| VP381 | 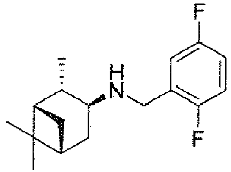 | 58.80 | 0.17 |
| VP390 | 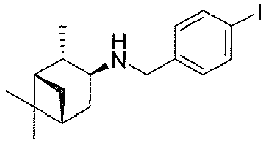 | 21.56 | 0.27 |
| VP391 | 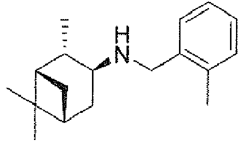 | 7.55 | 0.21 |
| VP393 | 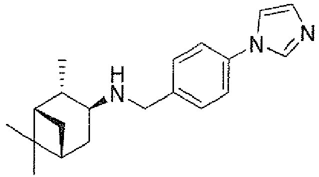 | 8.90 | 0.29 |
| VP396 | 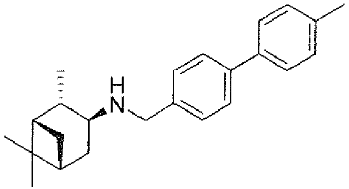 | 5.56 | 0.21 |
| VP397 | 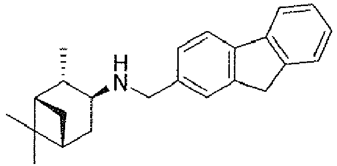 | 9.72 | 0.50 |

### Example 5: In vivo evaluation of molecules in a Leishmania donovani infantum infection model in mice

### Protocol: in vivo evaluation in mice

Compounds VP343, VP370 were evaluated *in vivo* for their antileishmanial properties by oral and intravenous route on the *Leishmania donovani*/Balb/c mouse model, comparatively to miltefosine, used as reference drug, according to a previously described protocol (Balaraman et al., 2015). Six- to eight- week-old Balb/c mice (Élevages Janvier, Le Genest Saint Isle, France) were infected intravenously on day 1 with 10⁷ *L. donovani* (MHOM/ET/67/HU3) amastigotes derived from the spleen of hamsters and randomly sorted into four groups of 10 mice and one group of 12 mice as infected controls but not treated. The treatment administered orally or intravenously, started one week post-infection, on day 8, and continued for 5 consecutive days. At day 15, all groups of mice were autopsied and livers and spleens were weighed. Parasite load in the liver was determined by counting the number of amastigotes/500 liver cells in Giemsa-stained impression smears prepared from the liver and applying Stauber's formula. Three researchers counted the slides independently and the results are expressed as the mean values ±SD. The parasite burden of treated groups and controls were compared using Student's t- test or the Kruskal-Wallis nonparametric analysis of variance test for comparing two groups. Significance was established for a P value < 0.05.

### Results: In vivo efficacy evaluation of VP343 and VP370 by oral route in mice

Results are presented in table III below and in figure 1.

**Table III. Reduction of parasite burden in the liver of mice infected and treated with VP343 and VP370, compared to miltefosine.**

| **Batch** | **Number of mice** | **Treatment (Oral route)** | **LDU (x10⁹) ±SD** | **% reduction of parasite burden in the liver** |
|---|---|---|---|---|
| A | 12 | Untreated infected mice | 1.23 ± 0.29 | 0 |
| B | 12 | Miltefosine (10 mg/kg/day x 5) | 0.19 ± 0.09 | 84.6 |
| C | 8 | VP343 (20 mg/kg/day x 5) | 0.36 ± 0.08 | 70.8 |
| D | 8 | VP343 (10 mg/kg/day x 5) | 0.49 ± 0.07 | 60.2 |
| E | 8 | VP370 (20 mg/kg/day x 5) | 0.46 ± 0.12 | 62.7 |

The results show that VP343 and VP370 are significantly active *in vivo* by oral route after a treatment at 20 mg/kg/day x 5 and VP343 at 10 mg/kg/day x 5 (Tukey's multiple comparisons test) and have activities comparable to that of the reference molecule Miltefosine.

### Results: In vivo toxicity evaluation of VP343 and VP370 by oral route in mice

Biochemical parameters relative to VP343 and VP370 toxicity in Balb/C female mice (18-20 g) after a single treatment by oral route at 100 mg/kg under a 0.1 mL volume (= 5 mL/kg). Serum were collected one week after the treatment. Excipient: Methylcellulose 1% + Tween 0.1%.

**Table IV. Kidney function and liver toxicity parameters from mice exposed to VP343 or VP370 by oral route.**

| **Compound** | **Number of mice** | **Serum creatinin µM** | **Serum urea µM** | **Serum AST U/L ± SD** | **Serum AST U/L ± SD** |
|---|---|---|---|---|---|
| VP343 | 5 | <18 | 5.9±1.2 | 391±72 | 169±34 |
| VP370 | 5 | <18 | 7.9±1.5 | 330±68 | 197±41 |
| Untreated | 5 | <18 | 6.5±1.3 | 367±70 | 184±33 |

The results indicate that renal function parameters (creatinin and urea) and liver toxicity parameters are not significantly different between treated-groups of mice and the untreated control group using the U-Rank test. Thus, VP343 and VP370 did not show toxicity after a single treatment by oral route at 100 mg/kg (dose 10 and 5 fold higher than the efficacy dose).
AST: aspartate amino transferase
ALT: alanine amino transferase

Dosages were performed with a Cobas 800 (Roche/Hitachi) apparatus.

### Results: In vivo efficacy evaluation of VP343 by intravenous route in mice

Results are presented in table V below and in figure 2.

**Table V. Reduction of parasite burden in the liver of mice infected and treated with VP343, compared to miltefosine.**

| **Batch** | **Number of mice** | **Treatment (intravenous route)** | **LDU (x10⁸) ±SD** | **% reduction of parasite burden in the liver** |
|---|---|---|---|---|
| Control | 10 | Untreated infected mice | 1.23 ± 0.29 | 0 |
| VP343 | 8 | 10 mg/kg/day x 5 | 0.19 ± 0.09 | 42.4 |
| Miltefosine | 8 | 10mg/kg/day x 5 | 0.36 ± 0.08 | 65.9 |

The results show that VP343 is significantly active *in vivo* by intravenous route after a treatment at 10 mg/kg/day x 5 (Dunn's multiple comparisons test) and has an activity close to that of the reference molecule Miltefosine.

### Example 6: Identification of metabolites

### Protocol for VP343 metabolites' identification

The metabolites' identification of VP343 was performed after incubation of the compound with hepatic microsomal preparations from mouse, dog and human origin. The assay was performed by mixing VP343 at 5; 4; 2 or 1 µM with CD1 mouse hepatic microsomes (reference MIC255030 Biopredic) at 0.5 mg /mL of protein, or Beagle dog hepatic microsomes (reference MIC257013 Biopredic) at 1 mg/mL of protein, or human hepatic microsomes (reference MIC159882 Biopredic) at 1 mg/mL of protein, together with 1 mM NADPH (reference N1630 Sigma Aldrich), a phase I reaction's cofactor, in a 0.1 M phosphate buffer pH 7.4. Samples were then incubated in a thermomixer® (Eppendorf) at 37°C and 300 rotations per minute. After 45 minutes of incubation, the reaction was stopped with acetonitrile (0.5 volume of the experimental incubation volume). The samples' supernatants were then separated from the microsomes by centrifugation at 20,000 g during 10 minutes (Sorvall ST 16, Thermo Fisher Scientific) before analysis by liquid chromatography coupled to mass spectrometry (LC-MS).

The analysis was performed in two steps: first, on a positive electrospray ionisation source using a Waters Acquity I class UPLC (Ultra Performance Liquid Chromatography) system coupled to a Waters triple quadrupole mass spectrometer (Xevo TQMS) and then on an orbitrap source using a Thermo Fisher UHPLC (Ultra High Performance Liquid Chromatography) system (Ultimate 3000) coupled to a Thermo Fisher orbitrap mass spectrometer (Q Exactive) to confirm the structures of the identified metabolites. VP343 and his metabolites were separated by the UPLC system with a six minutes gradient of acetonitrile containing 0.1% of formic acid in a BEH C18 column (1.7 µm, 2.1 x 50 mm, Waters) with a mobile phase flow rate of 0.6 mL/min.

In each analytical system, the identification was performed in two steps. The first step aimed to identify the metabolites by injecting the sample in full scan. This means that, in a define range mass (m/z 250 to 450), each ion present in the sample should be detected. Thanks to the m/z of the different ions, some structure hypothesis can be made after the full scan injections. Those structures were then confirmed by injecting the sample in a daughter scan (for the Waters system) or in PRM (parallel reaction monitoring, for the Thermo Fisher system) mode. By adding collision energy, the products ions of the targeted metabolites were monitored to validate the supposed metabolite's structure obtained after injecting the sample in full scan mode.

### Identification of active metabolites of VP343

The metabolization of VP343 was studied by incubating the compound with mouse, dog, and human hepatic microsomes. The concentration of VP343 in the microsomal preparations was measured at t = 0 and at t = 45 min by liquid chromatography-tandem mass spectrometry (LC-MS). About 5% to 50% of VP343 remained after 45 min of incubation with mouse microsomes according to experiments (Table VI and Figure 3, respectively). About 5% of VP343 remained with dog microsomes. About 40 to 55% remained with human microsomes. These results indicate that VP343 is metabolized into other molecule(s) to various degrees depending on microsome origin as a consequence of metabolization.

**Table VI. Initial and remaining VP343 concentrations after incubation with mouse, dog and human hepatic microsomes.**

| VP343 initial concentrations | | | | |
|---|---|---|---|---|
| t=0 | | 4 µM | 2 µM | 1 µM |

| VP343 residual concentrations | | | | |
|---|---|---|---|---|
| Mouse | t = 45 min | 0.25 µM | 0.09 µM | 0.08 µM |
| Dog | t = 45 min | 0.12 µM | 0.09 µM | 0.03 µM |
| Human | t = 45 min | 1.62 µM | 1.08 µM | 0.41 µM |

The ability of the metabolite(s) present in the supernatants to inhibit the development of intramacrophage amastigotes was then tested. Supernatants from the 45 min VP343 / microsomal incubations were added to macrophages infected with amastigotes. Supernatant volumes were adjusted in order to correspond to 80, 40 and 20 nM of VP343 at t = 0. Amastigotes in macrophages were counted by fluorescence microscopy after 72 h. The results of Figure 3 indicate that the microsomal supernatants from all three species were able to inhibit intramacrophage amastigotes in a dose dependent manner with an IC₅₀ of 40 nM, identical to the IC₅₀ of the parent molecule VP343 (Table II). Thus, the VP343 metabolite mixes present in the 45 min microsomal supernatants have the same capacity to inhibit intramacrophage amastigotes as VP343.

To further investigate the number and nature of the metabolites, supernatants from mouse, dog and human hepatic microsomes incubated with VP343 for 45 min were analyzed by LC-MS (*cf.* protocol). The results of Figure 4 show that 11 metabolites could be detected.

MS-MS analysis (*cf.* protocol) enabled to identify the chemical structures of the metabolites, four metabolites being determined without ambiguity (scheme 1).

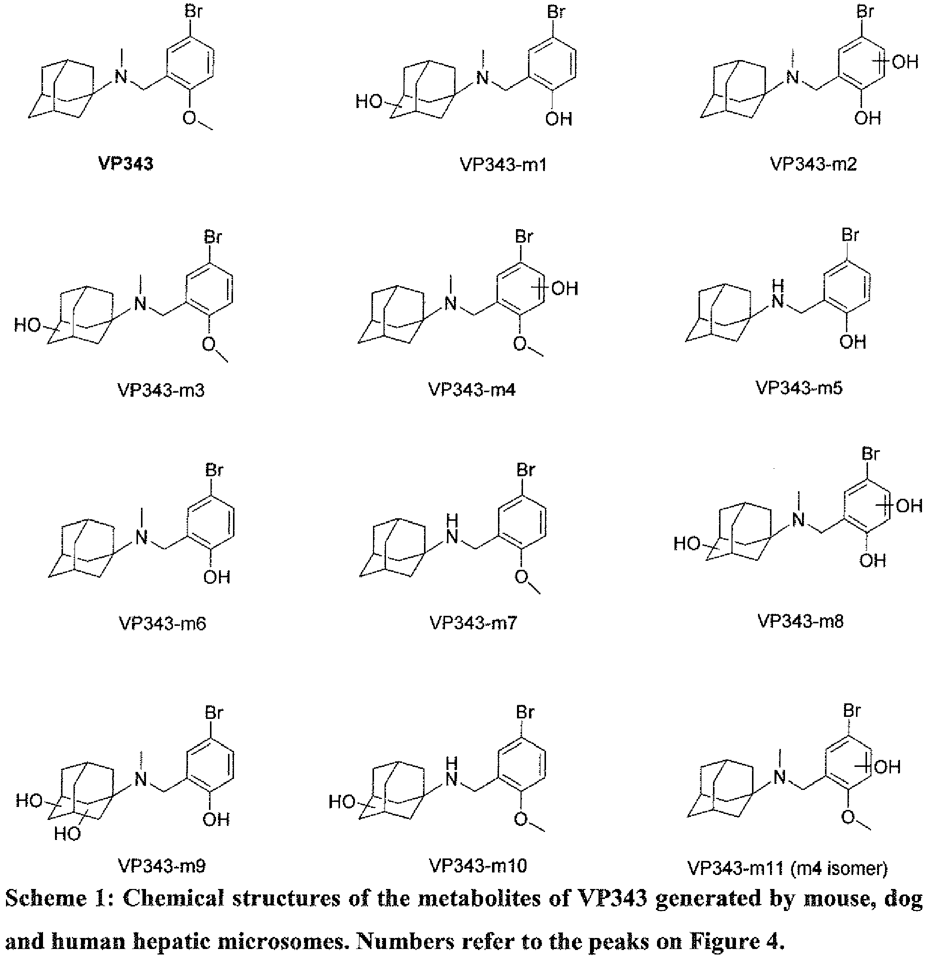

### Conclusion

The mixes of phase I VP343 metabolites generated by mouse, dog and human hepatic microsomes have the same activity as VP343 against intramacrophage amastigote leishmania parasites.

## Claims

1. A compound of formula (I) 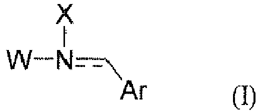 Wherein:
W is independently selected from:
- an adamantyl optionally substituted by one or more C₁-C₆ alkyl groups,
- a bicyclic monoterpene or a bicyclic monoterpenoid, in particular:
∘ a pinanyl,
∘ a bornanyl, or
∘ an isopinocamphenyl,
W being optionally substituted by one to three OH groups,
-̅-̅-̅-̅ represents a single bond or a double bond,
X is Ø or selected from H and C₁-C₆ alkyl groups, providing that:
- X is Ø when -̅-̅-̅-̅ represents a double bond, and
- X is selected from H and C₁-C₆ alkyl groups when -̅-̅-̅-̅ represents a single bond,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, OH, C₁-C₆ alkoxy, NO₂,
C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, OH, C₁-C₆ alkoxy and NO_{2,}
or said aryl or heteroaryl group Ar optionally forming with said aryl or heteroaryl group R a tricyclic compound, in particular a fluorenyl,
and the stereoisomeric forms, mixtures of stereoisomeric forms or pharmaceutically acceptable salts forms thereof,
for use in the treatment of leishmaniasis.

2. The compound of formula (I) for use of claim 1, to block or slow down the development of intramacrophagic amastigote *Leishmania* parasites.

3. The compound of formula (I) for use of claim 1, said compound being of one of the following formulae: 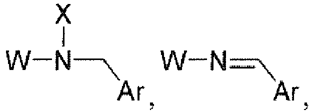 X being in particular H or Me.

4. The compound of formula (I) for use of any of claims 1 to 3, wherein Ar is a C₆-C₁₀ aryl, notably phenyl, or wherein Ar and a R group form together a fluorenyl, or wherein Ar is a 5 to 10 membered heteroaryl, notably a furanyl, an imidazolyl or an indolyl group.

5. The compound of formula (I) for use of any of claims 1 to 4, wherein R is selected from Br, Cl, I, methoxy, NO₂, imidazolyl and R'-phenyl, in particular p-methyl phenyl.

6. The compound of formula (I) for use of any of claims 1 to 5, wherein W is an adamantyl optionally substituted by one or more C₁-C₆ alkyl groups, in particular of the following formula: 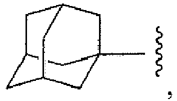 W being optionally substituted by one to three OH groups,
Ar being in particular a phenyl, being optionally substituted by one to three R groups, notably two R groups, in particular independently selected from F, Cl, Br, I, C₁-C₆ alkyl, C₁-C₆ alkoxy and NO₂, more particularly Br and methoxy.

7. The compound of formula (I) for use of any of claims 1 to 6, wherein W is a pinanyl, a bornanyl or an isopinocamphenyl, in particular of the following formula: 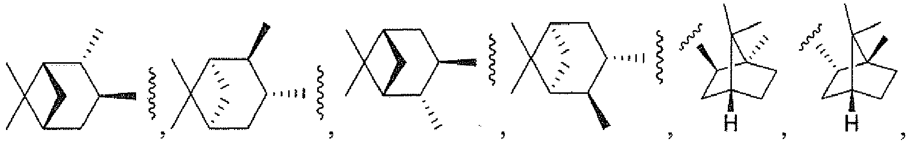 W being optionally substituted by one to three OH groups.

8. The compound of formula (I) for use of any of claims 1 to 7, providing that when Ar represents a phenyl substituted by an halogen, in particular Br, and a C₁-C₆ alkoxy, in particular a methoxy, more particularly a 5-bromo-2-methoxyphenyl group, then:
- -̅-̅-̅-̅ represents a double bond, or
- X represents a C₁-C₆ alkyl group, or
- W represents a pinanyl, a bornanyl or an isopinocamphenyl, in particular a pinanyl, W being optionally substituted by one to three OH groups.

9. The compound of formula (I) for use of claim 1, which is selected from:

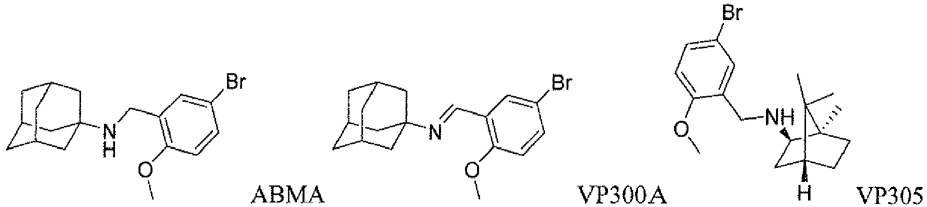

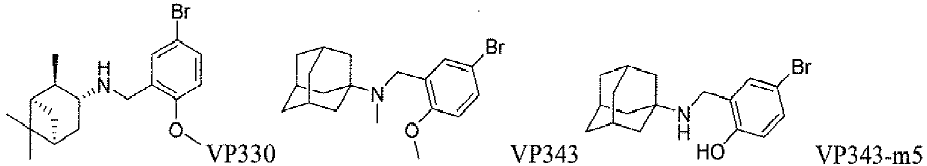

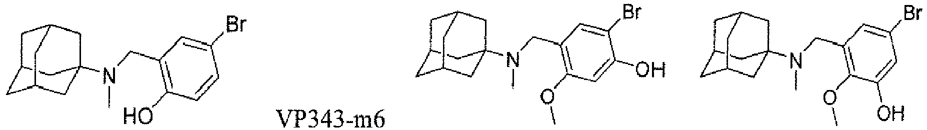

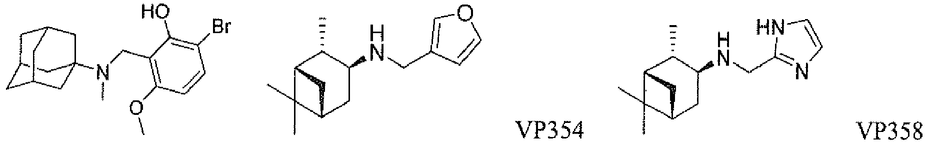

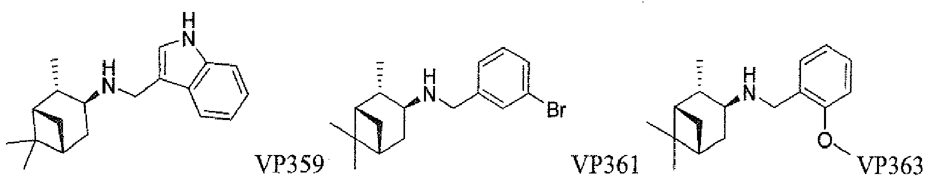

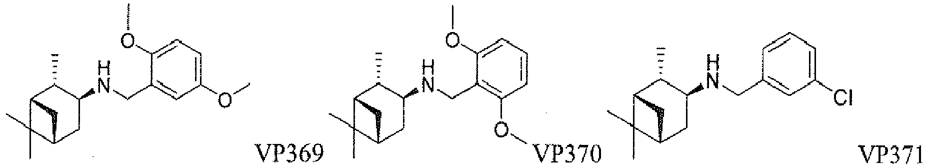

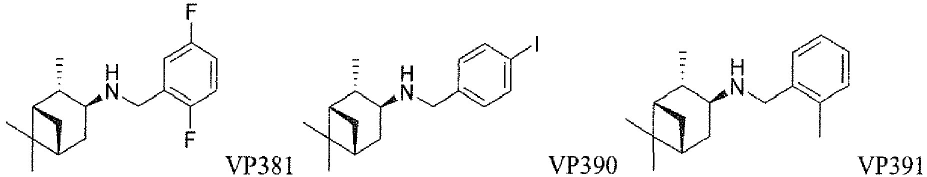

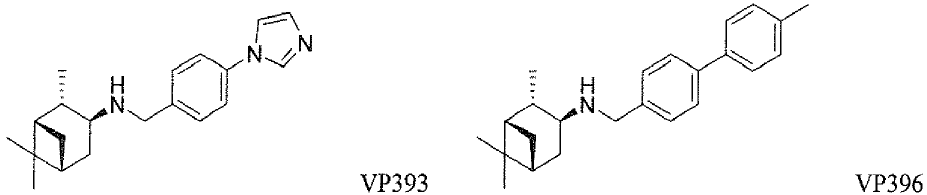

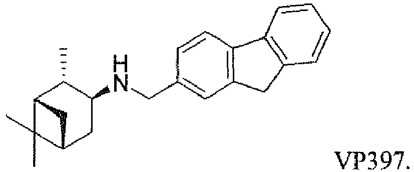

10. A pharmaceutical composition comprising a compound of formula (Ia): 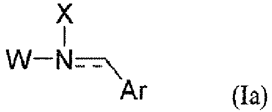 Wherein:
W is independently selected from:
- an adamantyl optionally substituted by one or more C₁-C₆ alkyl groups, or
- a pinanyl or an isopinocamphenyl, in particular 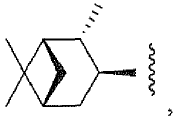 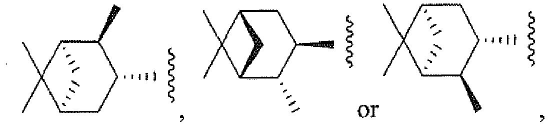
W being optionally substituted by one to three OH groups,
-̅-̅-̅-̅ represents a single bond or a double bond,
X is Ø or selected from H and C₁-C₆ alkyl groups, providing that:
- X is Ø when -̅-̅-̅-̅ represents a double bond, and
- X is selected from H and C₁-C₆ alkyl groups when -̅-̅-̅-̅ represents a single bond,
Ar is a C₆-C₁₀ aryl or a 5 to 10 membered heteroaryl, said aryl or heteroaryl groups being optionally substituted by one to three R groups,
R is independently selected from F, Cl, Br, I, C₁-C₆ alkyl, OH, C₁-C₆ alkoxy, NO_{2,}
C₆-C₁₀ aryl and 5 to 10 membered heteroaryl, said aryl or heteroaryl groups being optionally substituted by one to three R' groups,
R' is independently selected from F, Cl, Br, I, C₁-C₆ alkyl, OH, C₁-C₆ alkoxy and NO_{2,}
or said aryl or heteroaryl group Ar optionally forming with said aryl or heteroaryl group R a tricyclic compound, in particular a fluorenyl,
providing that:
- When W is an adamantyl optionally substituted by one or more C₁-C₆ alkyl groups, then:
∘ X is selected from C₁-C₆ alkyl groups, Ar being a phenyl substituted by an halogen, in particular Br, and a C₁-C₆ alkoxy, in particular a methoxy, or
∘ X is Ø, Ar being a phenyl substituted by an halogen, in particular Br, and a C₁-C₆ alkoxy, in particular a methoxy,
- When W is 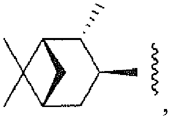 then:
∘ Ar is an indolyl optionally substituted by one to three R groups, or
∘ Ar is an substituted phenyl different from 5-bromo-2-methoxyphenyl, 5-iodo-2-methoxyphenyl, 4-iodophenyl and 4-fluorophenyl,
and the stereoisomeric forms, mixtures of stereoisomeric forms or pharmaceutically acceptable salts forms thereof,
in admixture with one or more pharmaceutically acceptable excipients.

11. The pharmaceutical composition of claim 10, wherein Ar is substituted by one to three R groups as defined in claim 1, at least one of them being a C₆-C₁₀ aryl or a 5 to 10 membered heteroaryl optionally substituted by one to three R' groups as defined in claim 1.

12. The pharmaceutical composition of claim 10, wherein said compound is selected from:

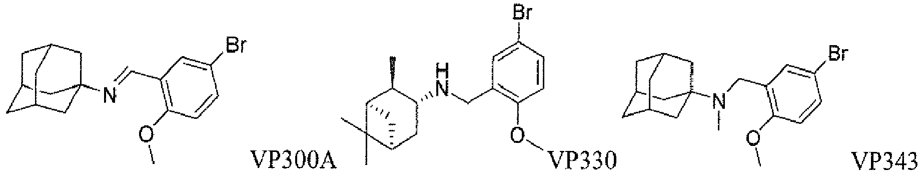

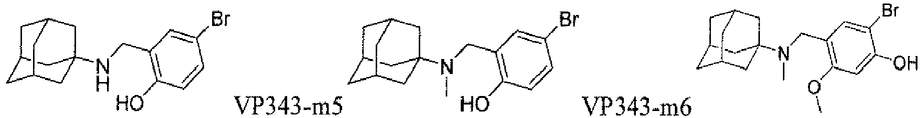

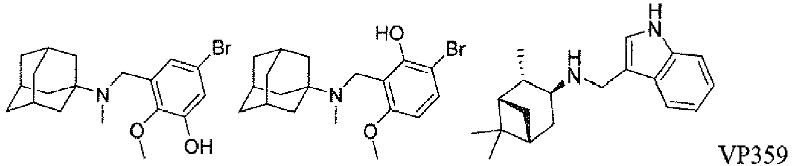

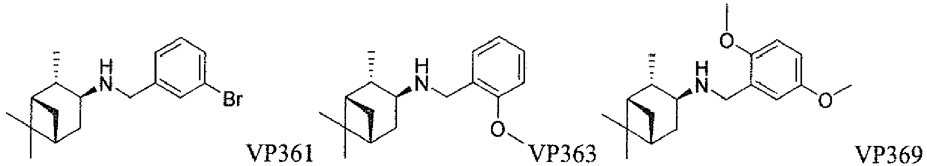

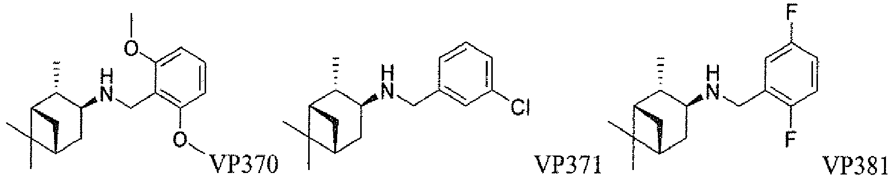

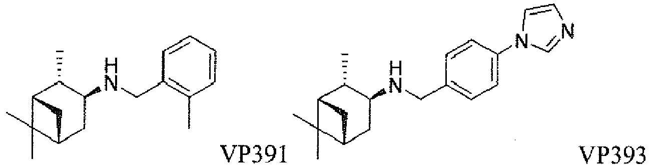

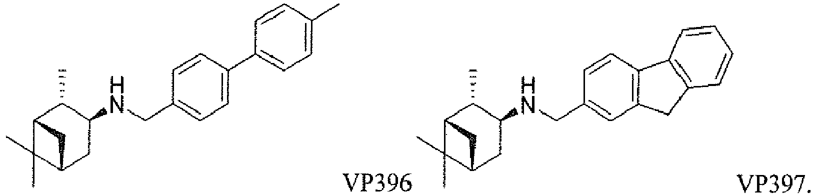

13. A compound of formula (Ia) as defined in any of claims 10 to 12, and the stereoisomeric forms, mixtures of stereoisomeric forms or pharmaceutically acceptable salts forms thereof.
